(19) Europäisches Patentamt
European Patent Office
Office européen des brevets

(11) Veröffentlichungsnummer: **0 111 746**
**B1**

(12) # EUROPÄISCHE PATENTSCHRIFT

(45) Veröffentlichungstag der Patentschrift: **16.09.87**

(51) Int. Cl.⁴: **C 07 D 311/16**, C 07 D 311/78, C 07 D 311/80, A 61 K 31/37

(21) Anmeldenummer: **83111339.4**

(22) Anmeldetag: **12.11.83**

(54) **Neue Sulfonsäureester von Hydroxycumarinen, ihre Herstellung und sie enthaltende Arzneimittel.**

(30) Priorität: **23.11.82 DE 3243158**

(43) Veröffentlichungstag der Anmeldung: **27.06.84 Patentblatt 84/26**

(45) Bekanntmachung des Hinweises auf die Patenterteilung: **16.09.87 Patentblatt 87/38**

(84) Benannte Vertragsstaaten: **AT BE CH DE FR GB IT LI NL SE**

(56) Entgegenhaltungen:
**CHEMICAL ABSTRACTS, Band 86, 1977, Seite 526, Nr. 121104w, Columbus, Ohio, US; I. DRAGOTA u.a.: "Potential anticancer agents. XII. Synthesis of new methanesulfonate derivatives from benzo- and naphthopyrans"**
**CHEMICAL ABSTRACTS, Band 52, 1958, Spalte 12854f, Columbus, Ohio, US; G.T. ESAYAN u.a.: "Esters of sulfonic acids. II. Synthesis of esters from 7-hydroxy-4-methylcoumarin and sulfonic acids"**
**CHEMICAL ABSTRACTS, Band 90, 1979, Seite 589, Nr. 151932q, Columbus, Ohio, US; V. NARAYANAN u.a.: "Deoxygenation of phenolic hydroxyl groups in coumarins"**

(73) Patentinhaber: **BASF Aktiengesellschaft, Carl-Bosch-Strasse 38, D-6700 Ludwigshafen (DE)**

(72) Erfinder: **Schlecker, Rainer, Dr., Suedring 8, D-6719 Bissersheim (DE)**
Erfinder: **Schmidt, Peter, Dr., Suedtiroler Ring 28, D-6719 Weisenheim (DE)**
Erfinder: **Thieme, Peter C., Dr., Dr.-Hans-Hoffmann-Strasse 5, D-6706 Wachenheim (DE)**
Erfinder: **Lenke, Dieter, Prof. Dr., Kekuleplatz 1, D-6700 Ludwigshafen (DE)**
Erfinder: **Teschendorf, Hans-Jürgen, Dr., Georg-Nuss-Strasse 5, D-6724 Dudenhofen (DE)**
Erfinder: **Traut, Martin, Dr., Muehltalstrasse 125, D-6900 Heidelberg (DE)**
Erfinder: **Mueller, Claus D., Dr., Odenwaldring 84, D-6806 Viernheim (DE)**
Erfinder: **Hofmann, Hans Peter, Dr., Untere Hart 12, D-6703 Limburgerhof (DE)**
Erfinder: **Kreiskott, Horst, Prof. Dr., Am Boehlig, D-6706 Wachenheim (DE)**

EP 0 111 746 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue Sulfonsäureester von Hydroxycumarinen, Verfahren zu ihrer Herstellung und diese Verbindungen als Wirkstoff enthaltende pharmazeutische Zubereitungen, die als wertvolle Arzneimittel bei der Behandlung von psychischen Störungen, insbesondere von Depressionen, verwendet werden können.

In verschiedenen Veröffentlichungen (C.A. 90, 1 519 329; 86 121 104 w; C.A. 52, 12 854f) ist eine Reihe von Sulfonsäureestern von 7-Hydroxy-4-methylcumarinen mit z.T. insektizider Wirkung oder schwacher Wirkung gegen Carcinosarcoma W256 beschrieben worden. Über eine antidepressive Aktivität dieser Verbindungen wurden jedoch keine Angaben gemacht.

Es wurde nun gefunden, dass Verbindungen der allgemeinen Formel I,

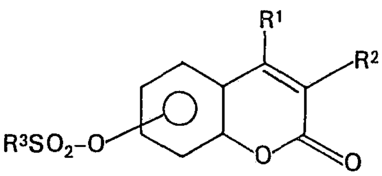

(I)

in der $R^1$ und $R^2$, die gleich oder verschieden sein können, für Wasserstoff, Halogen, vorzugsweise Chlor, einen Alkylrest von 1 bis 5 C-Atomen, der durch $-NR^4R^5$, $-OR^4$, $-CO_2R^4$ substituiert sein kann, wobei $R^4$ und $R^5$ gleich oder verschieden sind und ein Wasserstoffatom oder einen Alkylrest von 1 bis 4 C-Atomen bedeuten, oder für eine Carbonsäuregruppe $-CO_2R^4$ stehen, mit der Massgabe, dass $R^1$ nicht Wasserstoff oder Methyl ist, wenn $R^2$ Wasserstoff darstellt, oder zusammen eine $-(CH_2)_n$-Kette mit n = 3–5 bilden, und

$R^3$ für einen geradkettigen oder verzweigten Alkylrest mit 1 bis 8 C-Atomen oder einen Cycloalkylrest mit 3 bis 8 C-Atomen, die jeweils durch Halogen, $-OR^4$, $-NR^4R^5$, -CN oder einen Phenylring substituiert sein können; einen geradkettigen oder verzweigten Alkenylrest von 3 bis 8 C-Atomen; eine Aminogruppe $-NR^4R^5$ sowie für einen Phenyl- oder Naphthylrest steht, der gegebenenfalls ein- oder mehrfach substituiert ist durch $-OR^4$, $-NR^4R^5$, $-NO_2$, Halogen, $-SR^4$, $-SOR^4$, $-SO_2R^4$, $-SCF_3$, $-SO_2CF_3$, -CN, $-CO_2R^4$, $-COR^4$, $-NHCOR^4$, $-CF_3$, einen Alkylrest von $C_1$ bis $C_4$ oder Kombinationen dieser Substituenten, wertvolle pharmakologische Eigenschaften aufweisen. Die erfindungsgemässen Verbindungen der Formel I können hergestellt werden, indem man ein Hydroxycumarin der Formel II,

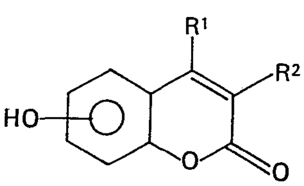

(II)

in der $R^1$ und $R^2$ die oben angegeben Bedeutungen haben, in an sich bekannter Weise mit einem Sulfonsäurederivat der Formel III,

$R^3SO_2X$ (III)

in der $R^3$ die für Formel I beschriebene Bedeutung hat und X für eine nucleofuge Abgangsgruppe wie Chlor, Brom oder $R^3SO_2O$ steht, umsetzt. Die Umsetzung kann, wie beispielsweise in Houben-Weyl, Georg Thieme-Verl. Stuttgart 1966, Bd. 9, S. 671–674 beschrieben, durch Erhitzen der beiden Komponenten, vorzugsweise in Anwesenheit eines inerten Lösungsmittels wie Benzol, Toluol, Methylenchlorid, Aceton, Ethanol, Dimethylformamid oder Wasser, durchgeführt werden. Die freiwerdende Säure wird im allgemeinen durch Zusatz von Basen wie Alkali- oder Erdalkalihydroxiden oder -carbonaten oder Aminen wie Dimethylanilin oder Pyridin abgefangen. Die eingesetzten Basen können im Überschuss auch als Lösungsmittel dienen. Anstelle der Hydroxycumarine der Formel II können auch deren Alkalimetallsalze mit den Sulfonsäurederivaten III, vorzugsweise unter wasserfreien Bedingungen in aprotischen Lösungsmitteln wie Ether, Tetrahydrofuran, Dimethylformamid (DMF) oder Dimethylsulfoxid (DMSO), umgesetzt werden. Als Basen können in diesen Fällen Alkalimetallhydride oder -alkoholate eingesetzt werden.

Weiterhin können Verbindungen der Formel I, in der $R^3$ einen substituierten aromatischen Rest bedeutet, nach bekannten Verfahren durch Einführung eines neuen oder Umwandlung eines schon vorhandenen Substituenten in $R^3$ hergestellt werden. Dazu bedient man sich der bekannten Methoden der Aromatenchemie. Beispielsweise lassen sich Hydroxycumarin-alkylsulfonylbenzolsulfonsäureester leicht durch Oxidation der entsprechenden Hydroxycumarin-alkylthiobenzolsulfonsäureester mit Oxydationsmitteln wie Wasserstoffperoxid oder Persäuren erhalten.

Die Hydroxycumarine II können nach bekannten Methoden, wie sie zum Beispiel in Elderfield R.C., Heterocyclic Compounds, John Wiley-Verl., New York 1951, Bd. 2, S. 174f beschrieben sind, hergestellt werden, beispielsweise durch Kondensation von Dihydroxybenzolen mit β-Ketocarbonsäureestern der Formel

$R^1-CO-CHR^2-CO_2C_2H_5$

in Anwesenheit eines Kondensationsmittels wie Schwefelsäure, Phosphorpentoxid oder Aluminiumchlorid.

Die Sulfonsäurederivate der Formel III sind überwiegend käuflich oder literaturbekannt und lassen sich nach bekannten Verfahren, wie sie in Houben-Weyl, Bd. 9, S. 389–398 und S. 547–599 zusammengestellt sind, herstellen.

Erfindungsgemässe Arzneimittelwirkstoffe, die nach den genannten Verfahren erhalten werden, sind beispielsweise die

Methan-, Ethan-, Propan-, Isopropan-, Butan-,
Isobutan-, sec-Butan-, tert.-Butan-, Pentan-,

1-Methylbutan-, 2-Methylbutan-, Isopentan-, 1,1-Dimethylpropan-, 2,2-Dimethylpropan-, Hexan-, Heptan-, Octan-, 2-Chlorethan-, 3-Chlorpropan-, 3-Chlorbutan-, Trifluormethan-, 2,2,2-Trifluorethan-, Trichlormethan-, 2-Methoxyethan-, 3-Methoxypropan-, 3-Methoxybutan-, 4-Methoxybutan-, 2-Ethoxyethan-, 2-Dimethylamino-ethan-, 3-Dimethylaminopropan-, 4-Dimethylaminobutan-, 3-Dimethylaminobutan-, 5-Dimethylaminopentan-, 3-Methylaminopropan-, Cyclopropan-, 1-Methylcyclopropan-, 2-Methylcyclopropan-, 2,2-Dimethylcyclopropan-, 1,2,2-Trimethylcyclopropan-, 2,2,3-Trimethylcyclopropan-, Cyclobutan-, 1-Methylcyclobutan-, 2-Methylcyclobutan-, 3-Methylcyclobutan-, 1-Propylcyclobutan-, 1-Butylcyclobutan-, Cyclopentan-, 1-Methylcyclopentan-, 2,5-Dimethylcyclopentan-, Cyclohexan-, 1-Methylcyclohexan-, Cycloheptan-, Cyclooctan-, Ethen-, Propen-(1)-, Propen-(2)-, Isopropen-, 1-Methylpropen-(1)-, 2-Methylpropen-(1)-, 1-Methylpropen-(2)-, Buten-(1)-, Buten-(2)-, Buten-(3)-, 1,2-Dimethylpropen-(1)-, 1-Ethylpropen-(1)-, 2-Methylbuten-(1)-, 3-Methylbuten-(1)-, 1-Methylbuten-(2)-, Penten-(1)-, Penten-(2)-, Penten-(3)-, Penten-(4)-, 1-Ethylbuten-(1)-, 2-Ethylbuten-(1)-, 2-Methylpenten-(1)-, 3-Methylpenten-(1)-, 3-Methylpenten-(2)-, Hexen-(3)-, Hexen-(5)-, 2-Ethyl-1-methylbuten-(1)-, Hepten-(1)-, Heptren-(6)-, Octen-(1)-, Octen-(7)-, 2-Toluol-, 3-Toluol-, 4-Toluol-, 3-Ethylbenzol-, 4-Ethylbenzol-, 4-Propylbenzol-, 2,3-Dimethylbenzol-, 2,4-Dimethylbenzol-, 2,5-Dimethylbenzol-, 2,6-Dimethylbenzol-, 3,4-Dimethylbenzol-, 3,5-Dimethylbenzol-, 2,4,6-Trimethylbenzol-, 2-Methoxybenzol-, 3-Methoxybenzol-, 4-Methoxybenzol-, 2-Ethoxybenzol-, 4-Ethoxybenzol-, 2,3-Dimethoxybenzol-, 2,4-Dimethoxybenzol-, 2,6-Dimethoxybenzol-, 3,4-Dimethoxybenzol-, 2,4,6-Trimethoxybenzol-, 2-Chlorbenzol-, 3-Chlorbenzol-, 4-Chlorbenzol-, 2-Brombenzol-, 3-Brombenzol-, 4-Brombenzol-, 2-Fluorbenzol-, 3-Fluorbenzol-, 4-Fluorbenzol-, 2-Nitrobenzol-, 3-Nitrobenzol-, 4-Nitrobenzol-, 2-Cyanobenzol-, 3-Cyanobenzol-, 4-Cyanobenzol-, 2-Methylthiobenzol-, 3-Methylthiobenzol-, 4-Methylthiobenzol-, 2-Ethylthiobenzol-, 3-Ethylthiobenzol-, 4-Ethylthiobenzol-, 2-Methylsulfonylbenzol-, 3-Methylsulfonylbenzol-, 4-Methylsulfonylbenzol-, 2-Methylsulfinylbenzol-, 4-Methylsulfinylbenzol-, 2-Acetylbenzol-, 3-Acetylbenzol-, 4-Acetylbenzol-, 3-Acetylaminobenzol-, 4-Acetylaminobenzol-, 2-Carbomethoxybenzol-, 3-Carbomethoxybenzol-, 4-Carbomethoxybenzol-, 2-Aminobenzol-, 3-Aminobenzol-, 4-Aminobenzol-, 3-Methylaminobenzol-, 4-Methylaminobenzol-, 3-Dimethylaminobenzol-, 4-Dimethylaminobenzol-, 2,3-Dichlorbenzol-, 2,4-Dichlorbenzol-, 2,5-Dichlorbenzol-, 2,6-Dichlorbenzol-, 3,4-Dichlorbenzol-, 3,5-Dichlorbenzol-, 2,4-Difluorbenzol-, 2,5-Difluorbenzol-, 2,6-Difluorbenzol-, 2-Chlor-6-fluorbenzol-, 3-Chlor-4-fluorbenzol-, 2-(Trifluormethyl)-benzol-, 3-(Trifluormethyl)-benzol-, 4-(Trifluormethyl)-benzol-, 2-Chlor-4-methylbenzol-, 2-Chlor-5-methyl-benzol-, 4-Chlor-2-methylbenzol-, 4-Chlor-3-methylbenzol-, 3-Chlor-4-methoxybenzol-, 4-Chlor-2-methoxybenzol-, 3-Fluor-4-methylbenzol-, 4-Fluor-3-methylbenzol-, 4-Chlor-2-(trifluormethyl)-benzol-, 4-Chlor-3-(trifluormethyl)-benzol-, 4-Fluor-3-(trifluormethyl)-benzol-, 4-Chlor-3-nitrobenzol-, 3-Chlor-4-nitrobenzol-, 3-Fluor-4-nitrobenzol-, 4-Nitro-3-(trifluormethyl)-benzol-, 1-Naphthalin-, 2-Naphthalinsulfonsäureester von 5-Hydroxy-3-methylcumarin, 6-Hydroxy-3-methylcumarin, 7-Hydroxy-3-methylcumarin, 8-Hydroxy-3-methylcumarin, 3,4-Dimethyl-5-hydroxycumarin, 3,4-Dimethyl-6-hydroxycumarin, 3,4-Dimethyl-7-hydroxycumarin, 3,4-Dimethyl-8-hydroxycumarin, 4-Ethyl-5-hydroxycumarin, 4-Ethyl-6-hydroxycumarin, 4-Ethyl-7-hydroxycumarin, 4-Ethyl-8-hydroxycumarin, 3-Ethyl-5-hydroxycumarin, 3-Ethyl-6-hydroxycumarin, 3-Ethyl-7-hydroxycumarin, 3-Ethyl-8-hydroxycumarin, 4-Ethyl-5-hydroxy-3-methylcumarin, 4-Ethyl-6-hydroxy-3-methylcumarin, 4-Ethyl-7-hydroxy-3-methylcumarin, 4-Ethyl-8-hydroxy-3-methylcumarin, 3-Ethyl-5-hydroxy-4-methylcumarin, 3-Ethyl-6-hydroxy-4-methylcumarin, 3-Ethyl-7-hydroxy-4-methylcumarin, 3-Ethyl-8-hydroxy-4-methylcumarin, 3,4-Diethyl-5-hydroxycumarin, 3,4-Diethyl-6-hydroxycumarin, 3,4-Diethyl-7-hydroxycumarin, 3,4-Diethyl-8-hydroxycumarin, 5-Hydroxy-4-methyl-3-propylcumarin, 6-Hydroxy-4-methyl-3-propylcumarin, 7-Hydroxy-4-methyl-3-propylcumarin, 8-Hydroxy-4-methyl-3-propylcumarin, 4-Ethyl-5-hydroxy-3-propylcumarin, 4-Ethyl-6-hydroxy-3-propylcumarin, 4-Ethyl-7-hydroxy-3-propylcumarin, 4-Ethyl-8-hydroxy-3-propylcumarin, 5-Hydroxy-3-methyl-4-propylcumarin, 6-Hydroxy-3-methyl-4-propylcumarin, 7-Hydroxy-3-methyl-4-propylcumarin, 8-Hydroxy-3-methyl-4-propylcumarin, 3-Ethyl-5-hydroxy-4-propylcumarin, 3-Ethyl-6-hydroxy-4-propylcumarin, 3-Ethyl-7-hydroxy-4-propylcumarin, 3-Ethyl-8-hydroxy-4-propylcumarin, 3,4-Dipropyl-5-hydroxycumarin, 3,4-Dipropyl-6-hydroxycumarin, 3,4-Dipropyl-7-hydroxycumarin, 3,4-Dipropyl-8-hydroxycumarin, 4-Butyl-5-hydroxy-3-methylcumarin, 4-Butyl-6-hydroxy-3-methylcumarin, 4-Butyl-7-hydroxy-3-methylcumarin, 4-Butyl-8-hydroxy-3-methylcumarin, 4-Butyl-3-ethyl-5-hydroxycumarin, 4-Butyl-3-ethyl-6-hydroxycumarin, 4-Butyl-3-ethyl-7-hydroxycumarin, 4-Butyl-3-ethyl-8-hydroxycumarin, 5-Hydroxy-3-methyl-4-pentylcumarin, 6-Hydroxy-3-methyl-4-pentylcumarin, 7-Hydroxy-3-methyl-4-pentylcumarin, 8-Hydroxy-3-methyl-4-pentylcumarin, 5-Hydroxy-4-methyl-3-pentylcumarin, 6-Hydroxy-4-methyl-3-pentylcumarin, 7-Hydroxy-4-methyl-3-pentylcumarin, 8-Hydroxy-4-methyl-3-pentylcumarin, 5-Hydroxy-3,4-trimethylencumarin, 6-Hydroxy-3,4-trimethylencumarin, 7-Hydroxy-3,4-trimethylencumarin, 8-Hydroxy-3,4-trimethylencumarin, 5-Hydroxy-3,4-tetramethylencumarin, 6-Hydroxy-3,4-tetramethylencumarin, 7-Hydroxy-3,4-tetramethylencumarin,

8-Hydroxy-3,4-tetramethylencumarin, 5-Hydroxy-3,4-pentamethylencumarin, 6-Hydroxy-3,4-pentamethylencumarin, 7-Hydroxy-3,4-pentamethylencumarin, 8-Hydroxy-3,4-pentamethylencumarin, 5-Hydroxy-4-methylcumarin-3-carbonsäuremethylester, 6-Hydroxy-4-methylcumarin-3-carbonsäuremethylester, 7-Hydroxy-4-methylcumarin-3-carbonsäuremethylester, 8-Hydroxy-4-methylcumarin-3-carbonsäuremethylester, 5-Hydroxy-3-methoxymethyl-4-methylcumarin, 6-Hydroxy-3-methoxymethyl-4-methylcumarin, 7-Hydroxy-3-methoxymethyl-4-methylcumarin, 8-Hydroxy-3-methoxymethyl-4-methylcumarin, 3-(2′-Dimethylaminoethyl)-5-hydroxy-4-methylcumarin, 3-(2′-Dimethylaminoethyl)-6-hydroxy-4-methylcumarin, 3-(2′-Dimethylaminoethyl)-7-hydroxy-4-methylcumarin, 3-(2′-Dimethylaminoethyl)-8-hydroxycumarin, 3-(3′-Dimethylaminopropyl)-7-hydroxy-4-methylcumarin, 3-(3′-Dimethylaminopropyl)-6-hydroxy-4-methylcumarin, 5-Hydroxy-3-chlorcumarin, 6-Hydroxy-3-chlorcumarin, 7-Hydroxy-3-chlorcumarin, 8-Hydroxy-3-chlorcumarin, 5-Hydroxy-4-chlorcumarin, 6-Hydroxy-4-chlorcumarin, 7-Hydroxy-4-chlorcumarin, 8-Hydroxy-4-chlorcumarin, 5-Hydroxy-3,4-dichlorcumarin, 6-Hydroxy-3,4-dichlorcumarin, 7-Hydroxy-3,4-dichlorcumarin, 8-Hydroxy-3,4-dichlorcumarin, 5-Hydroxy-3-chlor-4-methylcumarin, 6-Hydroxy-3-chlor-4-methylcumarin, 7-Hydroxy-3-chlor-4-methylcumarin, 8-Hydroxy-3-chlor-4-methylcumarin, 5-Hydroxy-4-chlor-3-methylcumarin, 6-Hydroxy-4-chlor-3-methylcumarin, 7-Hydroxy-4-chlor-3-methylcumarin, 8-Hydroxy-4-chlor-3-methylcumarin, 5-Hydroxy-3-fluorcumarin, 6-Hydroxy-3-fluorcumarin, 7-Hydroxy-3-fluorcumarin, 8-Hydroxy-3-fluorcumarin, 5-Hydroxy-4-fluorcumarin, 6-Hydroxy-4-fluorcumarin, 7-Hydroxy-4-fluorcumarin, 8-Hydroxy-4-fluorcumarin, 5-Hydroxy-3,4-difluorcumarin, 6-Hydroxy-3,4-difluorcumarin, 7-Hydroxy-3,4-difluorcumarin, 8-Hydroxy-3,4-difluorcumarin, 5-Hydroxy-3-fluor-4-methylcumarin, 6-Hydroxy-3-fluor-4-methylcumarin, 7-Hydroxy-3-fluor-4-methylcumarin, 8-Hydroxy-3-fluor-4-methylcumarin, 5-Hydroxy-4-fluor-3-methylcumarin, 6-Hydroxy-4-fluor-3-methylcumarin, 7-Hydroxy-4-fluor-3-methylcumarin, 8-Hydroxy-4-fluor-3-methylcumarin, 5-Hydroxy-3-bromcumarin, 6-Hydroxy-3-bromcumarin, 7-Hydroxy-3-bromcumarin, 8-Hydroxy-3-bromcumarin, 5-Hydroxy-4-bromcumarin, 6-Hydroxy-4-bromcumarin, 7-Hydroxy-4-bromcumarin, 8-Hydroxy-4-bromcumarin, 5-Hydroxy-3,4-dibromcumarin, 6-Hydroxy-3,4-dibromcumarin, 7-Hydroxy-3,4-dibromcumarin, 8-Hydroxy-3,4-dibromcumarin, 5-Hydroxy-3-chlor-4-methylcumarin, 6-Hydroxy-3-brom-4-methylcumarin, 7-Hydroxy-3-brom-4-methylcumarin, 8-Hydroxy-3-brom-4-methylcumarin, 5-Hydroxy-4-brom-3-methylcumarin, 6-Hydroxy-4-brom-3-methylcumarin, 7-Hydroxy-4-brom-3-methylcumarin, 8-Hydroxy-4-brom-3-methylcumarin, .

Schliesslich betrifft die Erfindung Arzneimittel, welche neben üblichen Träger- und Verdünnungsmitteln eine Verbindung der Formel I als Wirkstoff enthalten.

Die erfindungsgemässen Verbindungen weisen wertvolle pharmakologische Eigenschaften auf. Sie eignen sich zur Pharmakotherapie von psychischen Störungen, insbesondere als Antidepressiva mit hoher Wirkung. Sie können in üblicher Weise, z.B. oral oder intravenös, verabfolgt werden. Die Dosierung hängt vom Alter, Zustand und Gewicht des Patienten sowie von der Applikationsart ab. In der Regel beträgt die tägliche Wirkstoffdosis zwischen etwa 5 und 100 mg bei oraler Gabe.

Die Wirkqualitäten der erfindungsgemässen Substanzen wurden mit folgenden Tests erfasst:

Antidepressive Wirkung

Reserpin (2,15 mg/kg subkutan) verursacht an Mäusen (Stamm: Swiss; männlich, 20 bis 26 g Gewicht) eine Senkung der Körpertemperatur um durchschnittlich 3°C, gemessen 2 Stunden nach Applikation und bei einer Umgebungstemperatur von 20 bis 22°C. Antidepressiva hemmen diese Hypothermie dosisabhängig. Die Prüfsubstanzen werden oral 60 Minuten vor Reserpin appliziert.

Als $ED_{50}$ wird aus der linearen Regression zwischen log-Dosis (mg/kg) und relativer Abnahme der Hypothermie die Dosis ermittelt, welche die durch Reserpin induzierte Hypothermie um 50% hemmt.

Hemmung der Monoaminoxidase

Die Bestimmung der Monoaminoxidase A erfolgt in verdünntem Rattenhirnhomogenat, dem 1. unterschiedliche Konzentrationen der zu prüfenden Testsubstanzen und 2. $^{14}C$-Tryptamin in einer Konzentration von 0,4 µmol/l zugesetzt werden. Dieser Ansatz wird 20 Minuten bei 37°C inkubiert. Die Reaktion wird dann durch 0,1 n wässrige HCl gestoppt und die Reaktionsprodukte nach Extraktion mit Toluol-Szintillator (PPO + POPOP in Toluol) bestimmt. Der Blindwert wird in analogen Ansätzen mit einer Inkubationszeit von t = 0 Minuten bestimmt.

Aus den bei den verschiedenen Inhibitorkonzentrationen gegen die Kontrolle ermittelten Hemmwerten wird durch lineare Regression nach logit-log-Transformation die mittlere Hemmkonzentration ($IC_{50}$) berechnet.

Die im Reserpin-Test gefundenen Ergebnisse sind in Tabelle 1 zusammengestellt.

Für alle in der Tabelle angeführten 19 Beispiele fanden sich dosisabhängige Wirkungen mit $ED_{50}$-Werten zwischen 0,3 (Beispiel 47) und 9,0 mg (Beispiel 30). Der Grossteil der gefundenen ED-Werte liegt unter 4 mg; die Substanzen sind damit als hochwirksam zu bezeichnen.

Als Wirkungsmechanismus für die Substanzen wurde eine Hemmung der Monoaminoxidase A gefunden, ein Wirkungsmechanismus, wie er von der Vergleichssubstanz Pargylin bekannt ist. Für Pargylin wird im Reserpintest als Mass für die antidepressive Wirkung eine $ED_{50}$ von 48 mg gefun-

den (s. Tab. 1). Ein Vergleich der angeführten Beispiele mit Pargylin zeigt, dass alle 19 Beispiele wirksamer sind als Pargylin, davon 16 mehr als 10fach.

Die Werte für die Hemmung der Monoaminoxidase A sind in Tabelle 2 zusammengestellt.

Für alle Beispiele in Tab. 2 finden sich dosisabhängige Hemmwirkungen gegenüber der Monoaminoxidase A, so dass für jede Substanz eine $IC_{50}$ angegeben werden kann. Diese $IC_{50}$-Werte liegen in einem Bereich von maximal 0,7 (Beispiel 33) bis minimal 0,003 (Beispiel 10) µmol/l. Bei der Mehrzahl der Substanzen liegen die gefundenen $IC_{50}$-Werte unter 0,1 µmol/l. Alle Substanzen sind wirksamer (3 bis 666mal) als Pargylin, das in diesem Testmodell eine $IC_{50}$ von 2,0 µmol/l aufweist.

Aufgrund der pharmakologischen Befunde sind die erfindungsgemässen Substanzen in entsprechender galenischer Zubereitung für die Pharmakotherapie von psychischen Erkrankungen, insbesondere von Depressionen, geeignet.

Tabelle 1

| Beispiel Nr. | Antidepressive Wirkung (Maus) $ED_{50}$ mg/kg p.o. |
|---|---|
| 1 | 1,2 |
| 2 | 5,9 |
| 4 | 4,0 |
| 9 | 6,1 |
| 11 | 2,5 |
| 14 | 1,4 |
| 18 | 3,6 |
| 22 | 1,4 |
| 23 | 3,1 |
| 24 | 3,7 |
| 26 | 1,8 |
| 30 | 9,0 |
| 38 | 1,4 |
| 39 | 1,7 |
| 40 | 1,9 |
| 47 | 0,3 |
| 49 | 1,3 |
| 50 | 3,4 |
| 51 | 1,5 |
| Pargylin | 48 |

Tabelle 2

| Beispiel Nr. | Monoaminoxidase Hemmung $IC_{50}$ µmol/l |
|---|---|
| 1 | 0,024 |
| 2 | 0,11 |
| 3 | 0,076 |
| 4 | 0,080 |
| 5 | 0,016 |
| 6 | 0,11 |
| 7 | 0,021 |
| 8 | 0,16 |
| 9 | 0,0050 |
| 10 | 0,0029 |
| 11 | 0,0060 |

Tabelle 2 (Fortsetzung)

| Beispiel Nr. | Monoaminoxidase Hemmung $IC_{50}$ µmol/l |
|---|---|
| 12 | 0,051 |
| 13 | 0,027 |
| 14 | 0,0077 |
| 15 | 0,62 |
| 16 | 0,039 |
| 17 | 0,056 |
| 18 | 0,0080 |
| 19 | 0,0098 |
| 20 | 0,067 |
| 21 | 0,55 |
| 22 | 0,0096 |
| 23 | 0,0037 |
| 24 | 0,075 |
| 25 | 0,077 |
| 26 | 0,0073 |
| 27 | 0,034 |
| 28 | 0,29 |
| 29 | 0,11 |
| 30 | 0,0063 |
| 31 | 0,030 |
| 32 | 0,12 |
| 33 | 0,70 |
| 34 | 0,023 |
| 35 | 0,12 |
| 36 | 0,093 |
| 37 | 0,067 |
| 39 | 0,0084 |
| 41 | 0,077 |
| 42 | 0,11 |
| 43 | 0,022 |
| 45 | 0,54 |
| 46 | 0,54 |
| 47 | 0,0057 |
| 48 | 0,19 |
| 53 | 0,22 |
| 54 | 0,014 |
| Pargylin | 2,0 |

Die neuen Wirkstoffe können in den üblichen galenischen Applikationsformen fest oder flüssig angewendet werden, wie Tabletten, Kapseln, Pulver, Granulate, Dragees oder Lösungen. Diese werden in üblicher Weise hergestellt. Die Wirkstoffe können dabei mit den gebräuchlichen galenischen Hilfsmitteln (hauptsächlich Träger- und Verdünnungsmitteln) wie Talkum, Gummiarabikum, Saccharose, Lactose, Getreide- oder Maisstärke, Kartoffelmehl, Magnesiumstearat, Alginaten, Gummi-Tragacanth, Carraghenaten, Polyvinylalkohol, Polyvinylpyrrolidon, wässrigen oder nicht wässrigen Trägern, Netzmitteln, Dispergiermitteln, Emulgatoren und/oder Konservierungsmitteln verarbeitet werden (vgl. H. Sucker et al., Pharmazeutische Technologie, Thieme-Verl., Stuttgart 1978). Die so erhaltenen Präparate enthalten den Wirkstoff normalerweise in einer Menge von 0,1 bis 93 Gew.%.

Herstellung von Ausgangsverbindungen:

3,4-Dimethyl-7-hydroxycumarin

Zu 260 g konz. Schwefelsäure wurde bei 10°C in 3–4 Stunden eine Mischung von 58 g (0,53 Mol) Resorcin und 90 g (0,53 Mol) 2-Methylacetessigsäureethylester (85%) getropft. Die Mischung wurde über Nacht bei Raumtemperatur (RT) gerührt und auf 2000 g Eis gegossen. Der Niederschlag wurde abgesaugt und in 2 l Wasser suspendiert. Durch Zugabe von konz. Natronlauge wurde die Mischung stark alkalisch gestellt, filtriert und das Filtrat mit konz. HCl angesäuert. Der Niederschlag wurde abgesaugt, mit Wasser gewaschen und getrocknet. Ausbeute 85,2 g (85%), Fp. 258°C.

Nach dem gleichen Verfahren wurden beispielsweise die folgenden Verbindungen hergestellt:

7-Hydroxy-4-methyl-3-propylcumarin, Ausbeute 70%, Fp. 136°C

3-Ethyl-7-hydroxy-4-methylcumarin, Ausbeute 79%, Fp. 195°C

4-Ethyl-7-hydroxy-3-methylcumarin, Ausbeute 65%, Fp. 184°C

4-Ethyl-7-hydroxycumarin, Ausbeute 90%, Fp. 168°C

6-Hydroxy-3,4-dimethylcumarin, Ausbeute 14%, Fp. 239°C

7-Hydroxycumarin-3-essigsäureethylester

50 g (0,36 Mol) 2,4-Dihydroxybenzaldehyd und 58 g (0,36 Mol) Malonsäurediethylester wurden unter Kühlen mit 30 g (0,36 Mol) Piperidin versetzt und anschliessend 4 Stunden bei RT gerührt. Nach Zugabe von 500 ml Wasser wurde die Lösung mit konz. Salzsäure auf pH 8 gestellt, der Niederschlag abgesaugt, mit Wasser gewaschen und getrocknet, Ausbeute 38 g (45%), Fp. 168–170°C.

Herstellung erfindungsgemässer Wirkstoffe:

Beispiel 1

7-Hydroxy-3,4-dimethylcumarin-benzolsulfonsäureester

2,3 g 7-Hydroxy-3,4-dimethylcumarin wurden in 50 ml Pyridin gelöst und bei 20°C mit 4,7 g Benzolsulfochlorid versetzt. Die Mischung wurde über Nacht gerührt, auf 200 g Eis gegossen, der Niederschlag abgesaugt, mit Wasser gewaschen, aus Ethanol umkristallisiert und getrocknet. Ausbeute 3,3 g (83%), Fp. 177–178°C

$C_{17}H_{14}O_5S$ (330)

Ber. 61,8 C 4,3 H 24,2 O
Gef. 62,1 C 4,3 H 23,9 O

Nach dem gleichen Verfahren wurden hergestellt:

Beispiel 2

7-Hydroxy-3,4-dimethylcumarin-methansulfonsäureester

Ausbeute 69%, Fp. 185°C

$C_{12}H_{12}O_5S$ (268)
Ber. 53,7 C 4,5 H
Gef. 53,8 C 4,3 H

Beispiel 3

7-Hydroxy-3,4-dimethylcumarin-naphthalin-1′-sulfonsäureester

Ausbeute 91%, Fp. 144–146°C

$C_{21}H_{16}O_5S$ (380)
Ber. 66,3 C 4,2 H 8,4 S
Gef. 66,2 C 4,3 H 8,3 S

Beispiel 4

7-Hydroxy-3,4-dimethylcumarin-naphthalin-2′-sulfonsäureester

Ausbeute 68%, Fp. 135–136°C

$C_{21}H_{16}O_5S$ (380)
Ber. 66,3 C 4,2 H
Gef. 66,6 C 4,5 H

Beispiel 5

7-Hydroxy-3,4-dimethylcumarin-toluol-4′-sulfonsäureester

$C_{18}H_{16}O_5S$ (340)
Ber. 62,8 C 4,7 H 23,2 O
Gef. 62,7 C 4,7 H 23,4 O

Beispiel 6

7-Hydroxy-3,4-dimethylcumarin-2′-nitrobenzolsulfonsäureester

Ausbeute 23% (Ethanol/DMF), Fp. 187–189°C

$C_{17}H_{13}NO_7S$ (375)
Ber. 54,4 C 3,5 H 3,7 N
Gef. 54,5 C 3,6 H 3,8 N

Beispiel 7

7-Hydroxy-3,4-dimethylcumarin-4′-methoxybenzolsulfonsäureester

Ausbeute 82% (Ethanol/DMF), Fp. 138–140°C

$C_{18}H_{16}O_6S$ (360)
Ber. 60,0 C 4,3 H
Gef. 60,1 C 4,5 H

Beispiel 8

7-Hydroxy-3,4-dimethylcumarin-4′-(α,α,β,β-tetrafluorethoxy)-benzolsulfonsäureester

Ausbeute 78% (EtOH), Fp. 123–126°C

$C_{19}H_{14}F_4O_6S$ (446)
Ber. 51,1 C 3,2 H 7,2 F
Gef. 51,2 C 3,3 H 7,5 F

Beispiel 9

7-Hydroxy-3,4-dimethylcumarin-4′-chlorbenzolsulfonsäureester

Ausbeute 86% (Ethanol/DMF), Fp. 170–172°C

$C_{17}H_{13}ClO_5S$ (365)
Ber. 56,0 C 3,6 H 9,7 Cl
Gef. 54,7 C 3,5 H 3,7 N

Beispiel 10

7-Hydroxy-3,4-dimethylcumarin-4'-nitrobenzolsulfonsäureester

Ausbeute 67% (Ethanol/DMF), Fp. 200–203°C

$C_{17}H_{13}NO_7S$ (375)

| | | | |
|---|---|---|---|
| Ber. | 54,4 C | 3,5 H | 3,7 N |
| Gef. | 54,7 C | 3,5 H | 3,7 N |

Beispiel 11

7-Hydroxy-3,4-dimethylcumarin-4'-brombenzolsulfonsäureester

Ausbeute 82% (Ethanol/DMF), Fp. 162–168°C

$C_{17}H_{13}BrO_5S$ (409)

| | | | |
|---|---|---|---|
| Ber. | 49,9 C | 3,2 H | 19,5 Br |
| Gef. | 49,9 C | 3,2 H | 19,3 Br |

Beispiel 12

7-Hydroxy-3,4-dimethylcumarin-2',4',6'-trimethylbenzolsulfonsäureester

Ausbeute 82% (Ethanol/DMF), Fp. 188–190°C

$C_{20}H_{20}O_5S$ (372)

| | | | |
|---|---|---|---|
| Ber. | 64,5 C | 5,4 H | 21,5 O |
| Gef. | 64,5 C | 5,3 H | 21,6 O |

Beispiel 13

7-Hydroxy-3,4-dimethylcumarin-2',5'-dichlorbenzolsulfonsäureester

Ausbeute 83% (Ethanol/DMF), Fp. 178–180°C

$C_{17}H_{12}Cl_2O_5S$ (399)

| | | | |
|---|---|---|---|
| Ber. | 51,1 C | 3,0 H | 17,8 Cl |
| Gef. | 51,1 C | 3,0 H | 17,7 Cl |

Beispiel 14

7-Hydroxy-3,4-dimethylcumarin-4'-fluorbenzolsulfonsäureester

5,0 g 7-Hydroxy-3,4-dimethylcumarin in 20 ml DMF wurden bei 20°C zu einer Suspension von 1,15 g NaH (55%) in 50 ml DMF getropft. Nach 1 Std. wurde die Lösung mit 5,3 g 4-Fluor-benzolsulfochlorid versetzt, über Nacht bei Raumtemperatur gerührt und auf 500 g Eis gegossen. Der Niederschlag wurde abgesaugt, mit Wasser gewaschen, getrocknet und aus Ethanol/DMF umkristallisiert.

Ausbeute 6,7 g (74%), Fp. 162–165°C

$C_{17}H_{13}FO_5S$ (348)

| | | | |
|---|---|---|---|
| Ber. | 58,6 C | 3,8 H | 5,5 F |
| Gef. | 58,7 C | 3,9 H | 5,4 F |

Nach dem gleichen Verfahren wurden die folgenden Wirkstoffe hergestellt:

Beispiel 15

7-Hydroxy-3,4-dimethylcumarin-4'-acetylaminobenzolsulfonsäureester

Ausbeute 33%, Fp. 194–195°C

$C_{19}H_{17}NO_6S$ (387)

| | | | |
|---|---|---|---|
| Ber. | 59,9 C | 4,4 H | 24,8 O |
| Gef. | 58,8 C | 4,5 H | 24,6 C |

Beispiel 16

7-Hydroxy-3,4-dimethylcumarin-4'-methylthiobenzolsulfon-säureester

Ausbeute 32%, Fp. 129–131°C (Ethanol)

$C_{18}H_{16}O_5S_2$ (376)

| | | | |
|---|---|---|---|
| Ber. | 57,4 C | 4,3 H | 21,3 O |
| Gef. | 57,8 C | 4,4 H | 21,0 O |

Beispiel 17

7-Hydroxy-3,4-dimethylcumarin-4'-acetylbenzolsulfonsäureester

Ausbeute 49%, Fp. 157–159°C (Isopropanol/DMF)

$C_{19}H_{16}O_6S$ (372)

| | | | |
|---|---|---|---|
| Ber. | 61,3 C | 4,3 H | 25,8 O |
| Gef. | 61,5 C | 4,3 H | 25,7 O |

Beispiel 18

7-Hydroxy-3,4-dimethylcumarin-4'-cyanbenzolsulfonsäureester

Ausbeute 50%, Fp. 184–185°C (Ethanol/DMF)

$C_{18}H_{13}NO_5S$ (355)

| | | | |
|---|---|---|---|
| Ber. | 60,8 C | 3,7 H | 3,9 N |
| Gef. | 61,1 C | 3,9 H | 3,9 N |

Beispiel 19

7-Hydroxy-3-methylcumarin-4'-brombenzolsulfonsäureester

Ausbeute 67%, Fp. 210–212°C (Ethanol)

$C_{16}H_{11}BrO_5S$ (395)

| | | | |
|---|---|---|---|
| Ber. | 48,6 C | 2,8 H | 20,2 Br |
| Gef. | 48,8 C | 3,0 H | 20,0 Br |

Beispiel 20

7-Hydroxy-3-methylcumarin-benzolsulfonsäureester

Ausbeute 68%, Fp. 150–152°C (Ethanol)

$C_{16}H_{12}O_5S$ (316)

| | | |
|---|---|---|
| Ber. | 60,8 C | 3,8 H |
| Gef. | 61,0 C | 3,8 H |

Beispiel 21

7-Hydroxy-3,4-tetramethylencumarin-benzolsulfonsäureester

Ausbeute 89%, Fp. 185–186°C (Ethanol/DMF)

$C_{19}H_{16}O_5S$ (356)

| | | |
|---|---|---|
| Ber. | 64,0 C | 4,5 H |
| Gef. | 64,1 C | 4,6 H |

Beispiel 22

7-Hydroxy-3,4-dimethylcumarin-propansulfonsäureester

Ausbeute 64%, Fp. 107°C

$C_{14}H_{16}O_5S$ (296)

| | | |
|---|---|---|
| Ber. | 56,7 C | 5,4 H |
| Gef. | 56,8 C | 5,5 H |

Beispiel 23
7-Hydroxy-3,4-dimethylcumarin-isopropansulfonsäureester
Ausbeute 58%, Fp. 87°C

$C_{14}H_{16}O_5S$ (296)
Ber. 56,7 C 5,4 H
Gef. 56,5 C 5,6 H

Beispiel 24
7-Hydroxy-3,4-dimethylcumarin-trifluormethansulfonsäureester
Ausbeute 31%, Fp. 84°C (Ethanol)

$C_{13}H_9F_3SO_5$ (322)
Ber. 44,7 C 2,0 H 17,7 F
Gef. 44,8 C 3,0 H 16,8 F

Beispiel 25
7-Hydroxy-3,4-dimethylcumarin-phenylmethansulfonsäureester
Ausbeute 59%, Fp. 187–189°C (Ethanol/DMF)

$C_{18}H_{16}O_5S$ (344)
Ber. 62,8 C 4,7 H
Gef. 62,8 C 4,7 H

Beispiel 26
7-Hydroxy-3,4-dimethylcumarin-butansulfonsäureester
Ausbeute 56%, Fp. 101°C

$C_{15}H_{18}O_5S$ (310)
Ber. 58,1 C 5,9 H
Gef. 57,7 C 5,8 H

Beispiel 27
7-Hydroxy-3,4-dimethylcumarin-octansulfonsäureester
Ausbeute 49%, Fp. 65–67°C (Ethanol)

$C_{19}H_{26}O_5S$ (366)

Ber. 62,3 C 7,2 H 21,8 O
Gef. 62,2 C 7,0 H 21,9 O

Beispiel 28
7-Hydroxy-3,4-tetramethylencumarin-4'-brombenzolsulfonsäureester
Ausbeute 86%, Fp. 184–188°C

$C_{19}H_{15}BrO_5S$ (435)

Ber. 52,4 C 3,5 H 18,4 Br
Gef. 52,0 C 3,5 H 18,1 Br

Beispiel 29
7-Hydroxy-3,4-tetramethylencumarin-4'-chlorbenzolsulfonsäureester
Ausbeute 79%, Fp. 200–202°C

$C_{19}H_{15}ClSO_5$ (391)

Ber. 58,4 C 3,9 H 9,1 Cl
Gef. 58,7 C 3,9 H 8,7 Cl

Beispiel 30
7-Hydroxy-3-methylcumarin-4'-chlorbenzolsulfonsäureester
Ausbeute 91%, Fp. 190–193°C

$C_{16}H_{11}ClO_5S$ (351)
Ber. 54,8 C 3,2 H 10,1 Cl
Gef. 55,0 C 3,4 H 9,9 Cl

Beispiel 31
4-Ethyl-7-hydroxycumarin-4'-chlorbenzolsulfonsäureester
Ausbeute 66%, Fp. 155°C (Ethanol/DMF)

$C_{17}H_{13}ClO_5S$ (365)
Ber. 56,0 C 3,6 H 21,9 O 9,7 Cl
Gef. 55,9 C 3,8 H 21,7 O 9,8 Cl

Beispiel 32
4-Ethyl-7-hydroxycumarin-benzolsulfonsäureester
Ausbeute 69%, Fp. 108–109°C (Methanol)

$C_{17}H_{14}O_5S$ (330)
Ber. 61,8 C 4,3 H 24,2 O
Gef. 61,9 C 4,5 H 24,0 O

Beispiel 33
4-Ethyl-3-methyl-7-hydroxycumarin-benzolsulfonsäureester
Ausbeute 61%, Fp. 160–161°C (Ethanol/DMF)

$C_{18}H_{16}O_5S$ (344)
Ber. 62,8 C 4,7 H 23,2 O
Gef. 62,9 C 4,8 H 23,3 O

Beispiel 34
3-Ethyl-7-hydroxy-4-methylcumarin-4'-chlorbenzolsulfonsäureester
Ausbeute 58%, Fp. 196–197°C (Ethanol/DMF)

$C_{18}H_{15}ClO_5S$ (379)

Ber. 57,1 C 4,0 H 21,1 O 9,4 Cl
Gef. 57,1 C 4,0 H 21,1 O 9,4 Cl

Beispiel 35
3-Ethyl-7-hydroxy-4-methylcumarin-benzolsulfonsäureester
Ausbeute 65%, Fp. 147–148°C

$C_{18}H_{16}O_5S$ (344)

Ber. 62,8 C 4,7 H 23,2 O
Gef. 62,8 C 4,7 H 23,3 O

Beispiel 36
7-Hydroxy-4-methyl-3-propylcumarin-benzolsulfonsäureester
Ausbeute 63%, Fp. 124–125°C (Ethanol)

$C_{19}H_{18}O_5S$ (358)

Ber. 63,7 C 5,1 H 22,3 O
Gef. 64,1 C 5,1 H 22,1 O

Beispiel 37

7-Hydroxy-4-methyl-3-propylcumarin-4'-chlorbenzolsulfonsäureester

Ausbeute 46%, Fp. 133–134°C (Ethanol)

$C_{19}H_{17}ClO_5S$ (393)

Ber. 58,1 C 4,4 H 20,4 O 9,0 Cl

Gef. 58,4 C 4,5 H 20,1 O 8,9 Cl

Beispiel 38

7-Hydroxy-3,4-dimethylcumarin-pentansulfonsäureester

Ausbeute 44%, Fp. 72–74°C (Ethanol)

$C_{16}H_{20}O_5S$ (324)

Ber. 59,2 C 6,2 H 24,7 O

Gef. 58,7 C 6,2 H 24,4 O

Beispiel 39

7-Hydroxy-3,4-dimethylcumarin-ethansulfonsäureester

5,0 g 7-Hydroxy-3,4-dimethylcumarin wurden in 20 ml $H_2O$/1,1 g NaOH unter Rühren bei Raumtemperatur gelöst und mit 3,4 g Ethansulfochlorid versetzt. Die Mischung wurde über Nacht gerührt, der Niederschlag abgesaugt und aus Ethanol umkristallisiert.

Ausbeute 6,2 g (84%), Fp. 117–118°C

$C_{13}H_{14}O_5S$ (282)

Ber. 55,3 C 5,0 H 28,3 O

Gef. 55,1 C 5,0 H 28,5 O

Beispiel 40

7-Hydroxy-3,4-dimethylcumarin-N-methylamidosulfonsäureester

Ausbeute 26%, Fp. 146°C (Ethanol)

$C_{12}H_{13}NO_5S$ (283)

Ber. 50,9 C 4,6 H 4,9 N

Gef. 51,0 C 4,7 H 4,9 N

Beispiel 41

7-Hydroxy-3,4-dimethylcumarin-4'-methansulfonylbenzolsulfonsäureester

4,0 g 7-Hydroxy-3,4-dimethylcumarin-4'-methylthiobenzolsulfonsäureester wurden in 160 ml $CH_2Cl_2$/20 ml $CH_3OH$ gelöst und bei Raumtemperatur mit einer Lösung von 4,7 g 85% 3-Chlorperbenzoesäure in 50 ml $CH_2Cl_2$ versetzt. Die Lösung wurde über Nacht gerührt, das Lösungsmittel abgezogen, der Rückstand in $CH_2Cl_2$ gelöst und mehrmals mit $NaHCO_3$-Lösung gewaschen. Nach Trocknen über $Na_2SO_4$ wurde das Lösungsmittel abdestilliert und der Rückstand aus Ethanol/DMF umkristallisiert.

Ausbeute 1,7 g (38%), Fp. 227–228°C

$C_{18}H_{16}O_7S_2$ (408)

Ber. 52,9 C 4,0 H 27,1 O

Gef. 53,0 C 3,9 H 27,4 O

Beispiel 42

7-Hydroxy-3-carbomethoxymethyl-4-methylcumarinbenzolsulfonsäureester

Ausbeute 36%, Fp. 95–97°C

$C_{18}H_{14}O_7S$ (374)

Ber. 57,8 C 3,8 H

Gef. 57,5 C 4,0 H

Beispiel 43

7-Hydroxy-3-carbomethoxymethyl-4-methylcumarin-4'-brombenzolsulfonsäureester

Ausbeute 35%, Fp. 176–177°C (Ethanol/DMF)

$C_{18}H_{13}BrO_7S$ (453)

Ber. 47,7 C 2,9 H 17,6 Br

Gef. 47,9 C 3,2 H 17,0 Br

Beispiel 44

7-Hydroxy-3-carboethoxycumarin-benzolsulfonsäureester

Ausbeute 63%, Fp. 134–135°C (Ethanol)

$C_{18}H_{13}O_7S$ (374)

Ber. 57,8 C 3,8 H 29,9 O

Gef. 57,8 C 3,6 H 29,8 O

Beispiel 45

7-Hydroxy-3-carboethoxycumarin-4'-chlorbenzolsulfonsäureester

Ausbeute 72%, Fp. 168–169°C (Ethanol/DMF)

$C_{18}H_{13}ClO_7S$ (409)

Ber. 52,9 C 3,2 H 27,4 O 8,7 Cl

Gef. 52,9 C 3,3 H 27,2 O 8,6 Cl

Beispiel 46

7-Hydroxy-3-carboethoxycumarin-4'-nitrobenzolsulfonsäureester

Ausbeute 22%, Fp. 185–187°C (Ethanol/DMF)

$C_{18}H_{13}NO_9S$ (419)

Ber. 51,6 C 3,1 H 3,3 N

Gef. 51,8 C 3,2 H 3,5 N

Beispiel 47

7-Hydroxy-3,4-dimethylcumarin-2'-methylpropansulfonsäureester

Ausbeute 65%, Fp. 120°C (Ethanol)

$C_{15}H_{18}O_5S$ (310)

Ber. 58,1 C 5,9 H 25,8 O

Gef. 58,5 C 5,8 H 25,8 O

Beispiel 48

6-Hydroxy-3,4-dimethylcumarin-benzolsulfonsäureester

Ausbeute 40%, Fp. 147–150°C

$C_{17}H_{14}O_5S$ (330)

Ber. 61,8 C 4,3 H 24,2 O

Gef. 62,1 C 4,2 H 24,0 C

Beispiel 49
7-Hydroxy-3,4-dimethylcumarin-3′-chlorpropansulfonsäureester
Ausbeute 75%, Fp. 96–98°C (i-Propanol)

$C_{14}H_{15}ClO_5S$ (331)

| | | | | |
|---|---|---|---|---|
| Ber. | 50,8 C | 4,6 H | 24,2 O | 10,7 Cl |
| Gef. | 51,1 C | 4,6 H | 24,4 O | 10,2 Cl |

Beispiel 50
7-Hydroxy-3,4-dimethylcumarin-chlormethansulfonsäureester
Ausbeute 34%, Fp. 142–144°C (Ethanol)

$C_{12}H_{11}ClO_5S$ (302)

| | | | |
|---|---|---|---|
| Ber. | 48,2 C | 3,8 H | 11,4 Cl |
| Gef. | 47,7 C | 3,6 H | 11,8 Cl |

Beispiel 51
7-Hydroxy-3,4-dimethylcumarin-3′-nitrobenzolsulfonsäureester
Ausbeute 30%, Fp. 172–175°C (Ethanol)

$C_{17}H_{17}NO_7S$ (375)

| | | | |
|---|---|---|---|
| Ber. | 54,4 C | 3,5 H | 3,7 N |
| Gef. | 54,7 C | 3,6 H | 3,8 N |

Beispiel 52
7-Hydroxy-3,4-dimethylcumarin-4′-trifluormethylthiobenzolsulfonsäureester
Ausbeute 65%, Fp. 95–96°C (i-Propanol)

$C_{18}H_{13}F_3O_5S_2$ (430)

| | | | |
|---|---|---|---|
| Ber. | 50,2 C | 3,0 H | 13,2 F |
| Gef. | 50,5 C | 3,3 H | 13,2 F |

Beispiel 53
7-Hydroxy-3-chlor-4-methylcumarinbenzolsulfonsäureester
Ausbeute 44%, Fp. 143–146°C (i-Propanol)

$C_{16}H_{11}ClO_5S$ (351)

| | | | | |
|---|---|---|---|---|
| Ber. | 54,8 C | 3,2 H | 22,8 O | 10,1 Cl |
| Gef. | 55,1 C | 3,2 H | 22,8 O | 10,1 Cl |

Beispiel 54
7-Hydroxy-3-chlor-4-methylcumarin-isopropansulfonsäureester
Ausbeute 23%, Fp. 89–90°C (i-Propanol)

$C_{13}H_{13}ClO_5S$ (317)

| | | | | |
|---|---|---|---|---|
| Ber. | 49,3 C | 4,1 H | 25,3 O | 11,2 Cl |
| Gef. | 49,1 C | 4,0 H | 25,2 O | 11,7 Cl |

Beispiel 55
7-Hydroxy-3,4-dimethylcumarin-4′-trifluormethylsulfonylbenzolsulfonsäureester
16 g Substanz aus Beispiel 52 wurden wie im Beispiel 41 beschrieben oxidiert.
Ausbeute 9,1 g (53%), Fp. 170–173°C (Essigester)

$C_{18}H_{13}F_3O_7S_2$ (462)

| | | | |
|---|---|---|---|
| Ber. | 46,8 C | 2,8 H | 12,3 F |
| Gef. | 46,7 C | 3,0 H | 11,8 F |

**Patentansprüche für die Vertragsstaaten: BE, CH, DE, FR, GB, IT, LI, NL, SE**

1. Sulfonsäureester von Hydroxycumarinen der Formel I,

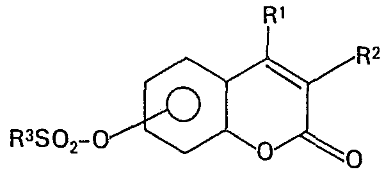

(I)

in der $R^1$ und $R^2$, die gleich oder verschieden sein können, für Wasserstoff, Halogen, einen Alkylrest von 1 bis 5 C-Atomen, der durch $-NR^4R^5$, $-OR^4$, $-CO_2R^4$ substituiert sein kann, wobei $R^4$ und $R^5$ gleich oder verschieden sind und ein Wasserstoffatom oder einen Alkylrest von 1 bis 4 C-Atomen bedeuten oder für einen Carboalkoxyrest $-CO_2R^4$ – stehen, mit der Massgabe, dass $R^1$ nicht Wasserstoff oder Methyl ist, wenn $R^2$ Wasserstoff darstellt, oder in der $R^1$ und $R^2$ zusammen eine $-(CH_2)_n$-Kette mit n = 3 bis 5 bilden, und
$R^3$ für einen geradkettigen oder verzweigten Alkylrest mit 1 bis 8 C-Atomen oder einen Cycloalkylrest mit 3 bis 8 C-Atomen, die jeweils durch Halogen, $-OR^4$, $-NR^4R^5$, CN oder einen Phenylring substituiert sein können, einen geradkettigen oder verzweigten Alkenylrest von 3 bis 8 C-Atomen; eine Aminogruppe $-NR^4R^5$ sowie für einen Phenyl- oder Naphthylrest steht, der gegebenenfalls ein- oder mehrfach substituiert ist durch
$-OR^4$, $-NR^4R^5$, $-NO_2$,
Halogen, $-SR^4$, $-S(O)R^4$, $-SO_2R^4$,
$-SCF_3$, $-SO_2CF_3$, -CN, $-C(O)R^4$, $-OC(O)R^4$,
$-NHC(O)R^4$, $-CF_3$, einen Alkylrest von $C_1$ bis $C_4$ oder Kombinationen dieser Substituenten.

2. Verbindungen der Formel I, in der $R^1$ und $R^2$ für Wasserstoff, Methyl, Ethyl oder Propyl und $R^3$ für einen Alkylrest mit 1 bis 8 C-Atomen, einen Fluoralkylrest mit 1 oder 2 C-Atomen, einen Cycloalkylrest mit 5 oder 6 C-Atomen oder einen ggf. gem. Anspruch 1 substituierten Phenylrest stehen, mit der Massgabe, dass $R^1$ nicht Wasserstoff oder Methyl ist, wenn $R^2$ Wasserstoff darstellt.

3. Verfahren zur Herstellung von Verbindungen nach Anspruch 1 oder 2, dadurch gekennzeichnet, dass man ein Hydroxycumarin der Formel II,

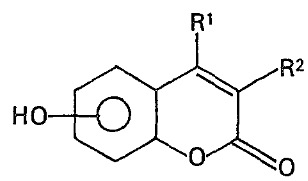

(II)

in der $R^1$ und $R^2$ die oben angegebenen Bedeutungen haben, in an sich bekannter Weise mit einem Sulfonsäurederivat der Formel III,

$R^3SO_2X$ (III)

in der $R^3$ die für Formel I beschriebene Bedeutung hat und X für eine nucleofuge Abgangsgruppe steht, in Gegenwart einer Base umsetzt.

4. Arzneimittel, das neben üblichen galenischen Hilfsmitteln eine Verbindung der Formel I nach Anspruch 1 oder 2 als Wirkstoff enthält.

5. Eine Verbindung der Formel I nach Anspruch 1 oder eine Verbindung der Formel I, in der $R^1$ Wasserstoff oder Methyl und $R^2$ Wasserstoff ist, zur Verwendung als Arzneimittel.

6. Eine Verbindung nach Anspruch 5 zur Verwendung als Arzneimittel bei der Behandlung depressiver Erkrankungen.

**Patentansprüche für den Vertragsstaat: AT**

1. Verfahren zur Herstellung von Sulfonsäureestern von Hydroxycumarinen der Formel I,

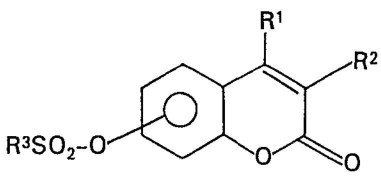

(I)

in der $R^1$ und $R^2$, die gleich oder verschieden sein können, für Wasserstoff, Halogen, einen Alkylrest von 1 bis 5 C-Atomen, der durch $-NR^4R^5$, $-OR^4$, $-CO_2R^4$ substituiert sein kann, wobei $R^4$ und $R^5$ gleich oder verschieden sind und ein Wasserstoffatom oder einen Alkylrest von 1 bis 4 C-Atomen bedeuten oder für einen Carboalkoxyrest $-CO_2R^4$ stehen, mit der Massgabe, dass $R^1$ nicht Wasserstoff oder Methyl ist, wenn $R^2$ Wasserstoff darstellt, oder in der $R^1$ und $R^2$ zusammen eine $-(CH_2)_n$-Kette mit n = 3 bis 5 bilden, und
$R^3$ für einen geradkettigen oder verzweigten Alkylrest mit 1 bis 8 C-Atomen oder einen Cycloalkylrest mit 3 bis 8 C-Atomen, die jeweils durch Halogen, $-OR^4$, $-NR^4R^5$, CN oder einen Phenylring substituiert sein können, einen geradkettigen oder verzweigten Alkenylrest von 3 bis 8 C-Atomen; eine Aminogruppe $-NR^4R^5$ sowie für einen Phenyl- oder Naphthylrest steht, der gegebenenfalls ein- oder mehrfach substituiert ist durch
$-OR^4$, $-NR^4R^5$, $-NO_2$,
Halogen, $-SR^4$, $-S(O)R^4$, $-SO_2R^4$,
$-SCF_3$, $SO_2CF_3$, $-CN$, $-C(O)R^4$,
$-OC(O)R^4$, $-NHC(O)R^4$, $-CF_3$,
einen Alkylrest von $C_1$ bis $C_4$ oder Kombinationen dieser Substituenten, dadurch gekennzeichnet, dass man ein Hydroxycumarin der Formel II,

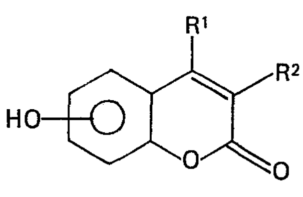

(II)

in der $R^1$ und $R^2$ die oben angegebenen Bedeutungen haben, in an sich bekannter Weise mit einem Sulfonsäurederivat der Formel III,

$R^3SO_2X$ (III)

in der $R^3$ die für Formel I beschriebene Bedeutung hat und X für eine nucleofuge Abgangsgruppe steht, in Gegenwart einer Base umsetzt.

2. Verfahren zur Herstellung von Verbindungen der Formel I nach Anspruch 1, wobei $R^1$ und $R^2$ für Wasserstoff, Methyl, Ethyl oder Propyl und $R^3$ für einen Alkylrest mit 1 bis 8 C-Atomen, einen Fluoralkylrest mit 1 oder 2 C-Atomen einen Cycloalkylrest mit 5 oder 6 C-Atomen oder einen ggf. gem. Anspruch 1 substituierten Phenylrest stehen, mit der Massgabe, dass $R^1$ nicht Wasserstoff oder Methyl ist, wenn $R^1$ Wasserstoff darstellt.

**Claims for the Contracting States: BE, CH, DE, FR, GB, IT, LI, NL, SE:**

1. A hydroxycoumarin sulfonate of the formula I

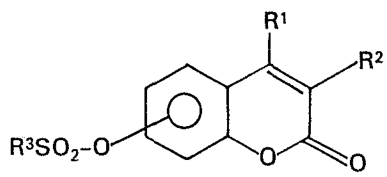

(I)

where $R^1$ and $R^2$, which may be identical or different, are each hydrogen, halogen, or alkyl of 1 to 5 carbon atoms which can be substituted by
$-NR^4R^5$, $-OR^4$ or $-CO_2R^4$,
where $R^4$ and $R^5$ are identical or different and are each hydrogen or alkyl of 1 to 4 carbon atoms, or $R^1$ and $R^2$ are each a carboalkoxy radical $-CO_2R^4$, with the proviso that $R^1$ is not hydrogen or methyl when $R^2$ is hydrogen, or $R^1$ and $R^2$ together form a $-(CH_2)_n$-chain where n is from 3 to 5, and
$R^3$ is a straight-chain or branched alkyl radical of 1 to 8 carbon atoms or cycloalkyl of 3 to 8 carbon atoms, each of which can be substituted by halogen, $-OR^4$, $-NR^4R^5$, $-CN$ or phenyl, or $R^3$ is a straight-chain or branched alkenyl radical of 3 to 8 carbon atoms, an amino group $-NR^4R^5$, or phenyl or naphthyl, each of which is unsubstituted or monosubstituted or polysubstituted by
$-OR^4$, $-NR^4R^5$, $-NO_2$,
halogen, $-SR^4$, $-S(O)R^4$, $-SO_2R^4$,
$-SCF_3$, $-SO_2CF_3$, $-CN$, $-C(O)R^4$,
$-OC(O)R^4$, $-NHC(O)R^4$, $-CF_3$, $C_1$-$C_4$-alkyl
or a combination of these substituents.

2. A compound of the formula I, where $R^1$ and $R^2$ are each hydrogen, methyl, ethyl or propyl, and $R^3$ is alkyl of 1 to 8 carbon atoms, fluoroalkyl of 1 or 2 carbon atoms, cycloalkyl of 5 or 6 carbon atoms or a phenyl radical which is unsubstituted or substituted according to claim 1, with the proviso that $R^1$ is not hydrogen or methyl when $R^2$ is hydrogen.

3. A process for the preparation of a compound as claimed in claim 1 or 2, wherein a hydroxycoumarin of the formula II

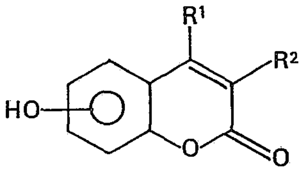

(II)

where $R^1$ and $R^2$ have the above meanings, is reacted in a conventional manner with a sulfonic acid derivative of the formula III

$R^3SO_2X$ (III),

where $R^3$ has the meanings given for formula I, and X is a nucleofugic leaving group, in the presence of a base.

4. A drug which contains, in addition to conventional pharmaceutical auxiliaries, a compound of the formula I as claimed in claim 1 or 2 as the active compounds.

5. A compound of the formula I as claimed in claim 1 or a compound of the formula I, where $R^1$ is hydrogen or methyl and $R^2$ is hydrogen, for use as a drug.

6. A compound as claimed in claim 5 for use as a drug in the treatment of depressive disorders.

**Claims for the Contracting State AT:**

1. A process for the preparation of a hydroxycoumarin sulfonate of the formula I

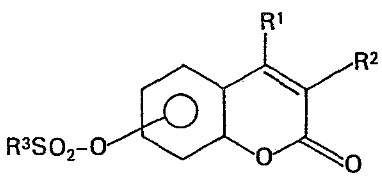

(I)

where $R^1$ and $R^2$, which may be identical of different, are each hydrogen, halogen, or alkyl of 1 to 5 carbon atoms which can be substituted by
$-NR^4R^5$, $-OR^4$ or $-CO_2R^4$,
where $R^4$ and $R^5$ are identical or $R^4$ et $R^5$ different each hydrogen or alkyl of 1 to 4 carbon atoms, or $R^1$ and $R^2$ are each a carboalkoxy radical $-CO_2R^4$, with the proviso that $R^1$ is not hydrogen or methyl when $R^2$ is hydrogen, or $R^1$ and $R^2$ together form a $-(CH_2)_n$-chain where n is from 3 to 5, and $R^3$ is a straight-chain or branched alkyl radical of 1 to 8 carbon atoms or cycloalkyl of 3 to 8 carbon atoms, each of which can be substituted by halogen, $-OR^4$, $-NR^4R^5$, -CN or phenyl, or $R^3$ is a straight-chain or branched alkenyl radical of 3 to 8 carbon atoms, an amino group $-NR^4R^5$, or phenyl or naphthyl, each of which is unsubstituted or monosubstituted or polysubstituted by
$-OR^4$, $-NR^4R^5$, $-NO_2$, halogen,
$-SR^4$, $-S(O)R^4$, $-SO_2R^4$, $-SCF_3$,
$-SO_2CF_3$, -CN, $-C(O)R^4$, $-OC(O)R^4$,
$-NHC(O)R^4$, $-CF_3$, $C_1$-$C_4$-alkyl
or a combination of these substituents, wherein a hydroxycoumarin of the formula II

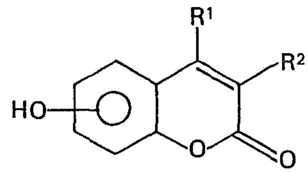

(II)

where $R^1$ and $R^2$ have the above meanings, is reacted in a conventional manner with a sulfonic acid derivative of the formula III

$R^3SO_2X$ (III),

where $R^3$ has the meanings given for formula I, and X is a nucleofugic leaving group, in the presence of a base.

2. A process for the preparation of a compound of the formula I as claimed in claim 1, where $R^1$ and $R^2$ are each hydrogen, methyl, ethyl or propyl, and $R^3$ is alkyl of 1 to 8 carbon atoms, fluoroalkyl of 1 or 2 carbon atoms, cycloalkyl of 5 or 6 carbon atoms or a phenyl radical which is unsubstituted or substituted according to claim 1, with the proviso that $R^1$ is not hydrogen or methyl when $R^2$ is hydrogen.

**Revendications pour les Etats contractants: BE, CH, DE, FR, GB, IT, LI, NL, SE**

1. Esters d'acide sulfonique et hydroxycoumarine de formule I

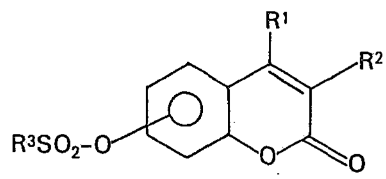

(I)

dans laquelle $R^1$ et $R^2$, qui peuvent être identiques ou différents, sont mis pour hydrogène, halogène, un reste alkyle de 1 à 5 atomes C, qui peut être substitué par
$-NR^4R^5$, $-OR^4$, $-CO_2R^4$,
étant identiques ou différents et représentant un atome d'hydrogène ou un reste alkyle de 1 à 4 atomes C, ou pour un reste carboalcoxy $-CO_2R^4$, sous réserve que $R^1$ n'est pas hydrogène ou méthyle lorsque $R^2$ représente hydrogène, ou bien dans laquelle $R^1$ et $R^2$ forment ensemble une chaîne $-(CH_2)_n$ avec n = 3 à 5, et
$R^3$ est mis pour un reste alkyle, à chaîne droite ou ramifiée, de 1 à 8 atomes C ou un reste cycloalkyle de 3 à 8 atomes C, qui peuvent être substitués par halogène, $-OR^4$, $-NR^4R^5$, CN ou un noyau phényle, pour un reste alcényle à chaîne droite ou ramifiée de 3 à 8 atomes C; pour un groupe amino $-NR^4R^5$, ainsi que pour un reste phényle ou naphtyle, qui est éventuellement substitué une ou plusieurs fois par
$-OR^4$, $-NR^4R^5$, $-NO_2$, halogène,
$-SR^4$, $-S(O)R^4$, $-SO_2R^4$, $SO_2CF_3$,
$-SCF_3$, -CN, $-C(O)R^4$, $-OC(O)R^4$, $-NHC(O)R^4$,

$-CF_3$, un reste alkyle de $C_1$ à $C_4$ ou des combinaisons de ces substituants.

2. Composés de formule I, dans laquelle $R^1$ et $R^2$ sont mis pour hydrogène, méthyle, éthyle ou propyle et $R^3$ pour un reste alkyle de 1 à 8 atomes C, un reste fluoralkyle de 1 à 2 atomes C, un reste cycloalkyle de 5 ou de 5 ou 6 atomes C ou un reste phényle éventuellement substitué selon la revendication 1, sous réserve que $R^1$ ne soit pas hydrogène ou méthyle lorsque $R^2$ représente hydrogène.

3. Procédé de préparation de composés selon la revendication 1 ou 2 caractérisé par le fait que l'on fait réagir, en présence d'une base, de manière connue en soi, une hydroxycoumarine de formule II

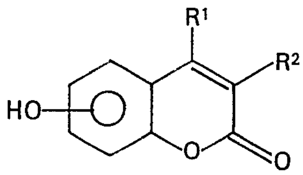

(II)

dans laquelle $R^1$ et $R^2$ ont les significations sus-indiquées, avec un dérivé d'acide sulfonique de formule III

$R^3SO_2X$ (III)

dans laquelle $R^3$ a la signification donnée pour la formule I et X représente un groupe éliminable nucléofuge.

4. Médicament qui, outre des auxiliaires galéniques, contient comme principe actif, un composé de formule I selon la revendication 1 ou 2.

5. Composé de formule I selon la revendication 1 ou composé de formule I dans laquelle $R^1$ représente hydrogène ou méthyle et $R^2$ hydrogène, pour utilisation comme médicament.

6. Composé selon la revendication 5 pour utilisation comme médicament pour le traitement de maladies dépressives.

**Revendications pour l'Etat contractant AT:**

1. Procédé de préparation d'esters d'acide sulfonique et hydroxycoumarine de formule I

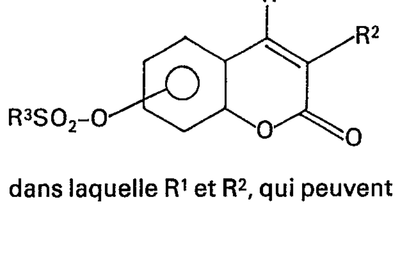

(I)

dans laquelle $R^1$ et $R^2$, qui peuvent être identiques ou différents, sont mis pour hydrogène, halogène, un reste alkyle de 1 à 5 atomes C, qui peut être substitué par

$-NR^4R^5$, $-OR^4$, $-CO_2R^4$, $R^4$ et $R^5$ étant identiques ou différents et représentant un atome d'hydrogène ou un reste alkyle de 1 à 4 atomes C, ou pour un reste carboalcoxy $-CO_2R^4$, sous réserve que $R^1$ n'est pas hydrogène ou méthyle lorsque $R^2$ représente hydrogène, ou bien dans laquelle $R^1$ et $R^2$ forment ensemble une chaîne $-(CH_2)_n$ avec $n = 3$ à 5, et

$R^3$ est mis pour un reste alkyle, à chaîne droite ou ramifiée, de 1 à 8 atomes C ou un reste cycloalkyle de 3 à 8 atomes C, qui peuvent être substitués par halogène, $-OR^4$, $-NR^4R^5$, CN ou un noyau phényle, pour un reste alcényle à chaîne droite ou ramifiée de 3 à 8 atomes C; pour un groupe amino $-NR^4R^5$, ainsi que pour un reste phényle ou naphtyle, qui est éventuellement substitué une ou plusieurs fois par

$-OR^4$, $-NR^4R^5$, $-NO_2$, halogène,
$-SR^4$, $-S(O)R^4$, $-SO_2R^4$,
$SO_2CF_3$, $-SCF_3$, $-CN$, $-C(O)R^4$,
$-OC(O)R^4$, $-NHC(O)R^4$, $-CF_3$, un reste alkyle de $C_1$ à $C_4$ ou des combinaisons de ces substituants, caractérisé par le fait que l'on fait réagir, en présence d'une base, de manière connue en soi, une hydroxycoumarine de formule II

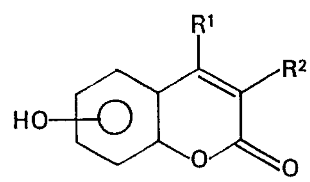

(II)

dans laquelle $R^1$ et $R^2$ ont les significations sus-indiquées, avec un dérivé d'acide sulfonique de formule III

$R^3SO_2X$ (III)

dans laquelle $R^3$ a la signification donnée pour la formule I et X représente un groupe éliminable nucléofuge.

2. Procédé de préparation de composés de formule I selon la revendication 1, $R^1$ et $R^3$ étant mis pour hydrogène, méthyle, éthyle ou propyle et $R^3$ pour un reste alkyle de 1 à 8 atomes C, un reste fluoralkyle de 1 à 2 atomes C, un reste cycloalkyle de 5 ou 6 atomes C ou un reste phényle éventuellement substitué selon la revendication 1, sous réserve que $R^1$ ne soit pas hydrogène ou méthyle lorsque $R^2$ représente hydrogène.